# EUROPEAN PATENT SPECIFICATION

(11) **EP 1 532 093 B1**
(45) Date of publication and mention of the grant of the patent: **29.03.2006**
(21) Application number: 03784005.5
(22) Date of filing: 02.07.2003
(51) Int. Cl.: C07C 37/60

(54) **HYDROXYLATION PROCESS FOR AROMATIC COMPOUNDS CONTAINING A DIOXA- HETEROCYCLIC SYSTEM**
HYDROXYLIERUNGSVERFAHREN FUER AROMATISCHE VERBINDUNGEN, DIE EIN DIOXAHETEROCYCLISCHES SYSTEM ENTHALTEN
PROCEDE D'HYDROXYLATION DE COMPOSES AROMATIQUES CONTENANT UN SYSTEME DIOXA- HETEROCYCLIQUE

(30) Priority: 26.07.2002 IT MI20021662
(43) Date of publication of application: 25.05.2005
(73) Proprietor: Borregaard Italia S.p.A., 20123 Milano (IT)
(72) Inventor: ANDREOTTI, Grazia, I-20052 Monza-Milano (IT); PANSERI, Pietro, I-24100 Bergamo (IT); MESSORI, Vittorio, I-20129 Milan (IT); MARIANI, Oliviero, I-24040 Madone-Bergamo (IT); BOGNINI, Giezi, I-24036 Ponte S. Pietro-Bergamo (IT)
(74) Representative: De Gregori, Antonella
(86) International application number: PCT/EP2003/007056
(87) International publication number: WO 2004/014827

(56) References cited:
- EP-A- 1 110 910
- US-A- 4 701 428
- US-A- 5 840 997
- A.K.SINHABABU AND R.T.BORCHARDT: "Aromatic Hydroxylation. Hydroxybenzaldehydes from Bromobenzaldehydes via Reaction of in Situ Generated, Lithiated alfa-Morpholinobenzyl Alkoxides with Nitrobenzene" JOURNAL OF ORGANIC CHEMISTRY, vol. 48, no. 12, 1983, pages 1941-1944, XP002258954

## Description

The present invention relates to a hydroxylation process of aromatic compounds containing a heterocyclic system.

More specifically, the present invention relates to a process for the hydroxylation of 1,3-benzodioxoles.

Even more specifically, the present invention relates to a process for the oxidation of 1,3-benzodioxole (MDB) to give 5-hydroxy-1,3-benzodioxole (5-hydroxy-MDB).

Processes are known in literature, for the oxidation of aromatic compounds containing a heterocyclic system, such as benzodioxoles, which give, for example, products such as 5-hydroxy-MDB, a product which is used in agriculture and in the pharmaceutical and cosmetic industry and as an intermediate for organic syntheses.

English patent GB 2,323,843, for example, describes a process for the preparation of 5-hydroxy-MDB which consists in the oxidation of heliotropin with hydrogen peroxide (H₂O₂) in the presence of formic acid, according to the Baeyer Villiger reaction. This process, however, has the disadvantage of using a costly raw material as it is of a natural origin and is not easily available due to the fact that heliotropin is extracted from the sassafras plant currently protected by laws for the protection of the environment.

Another known process for producing 5-hydroxy-MDB is described in Japanese patent application 56-834865 of 1979. This process starts from MDB with the formation of the acetyl derivative, methylenedioxyacetophenone, followed by Baeyer Villiger oxidation using peracids. This method, currently in use, has the disadvantage of creating problems of an environmental nature due to the use of Friedel Kraft catalysts, the presence of methylenedioxyacetophenone and the use of dangerous oxidants, such as peracetic acid, in the oxidation phase of the corresponding acetophenone. As a whole, this synthesis method is characterized by two distinct phases, each of which is marked by heavy reaction conditions due to the use of particularly polluting catalysts and particularly dangerous reagents which are not easy to handle.

Another process described in Japanese patent application 63-45756 of 1993, starts from the derivative 5-bromo-1,3-benzodioxole followed by transformation into a borane derivative, by means of the Grignard reaction, and subsequent oxidation of the borane derivative with H₂O₂. This process is extremely lengthy, involves the use of various reagents which are not easy to handle on an industrial scale, such as the Grignard reactive intermediate and metallic magnesium, and the use of the borane agent which must then be oxidized in a second phase to the corresponding 5-hydroxy-MDB. The various passages of this process are onerous from an economical point of view due to the cost of the various reagents and waste load to be subjected to special disposal.

Another process, described in Japanese patent application 52-13384 of 1976, comprises the use of 1,2,4-triphenol as raw material and its subsequent cyclization with methylene chloride in a solvent medium, such as dimethylsulfoxide or dimethylformamide to obtain 5-hydroxy-MDB. This process has the disadvantage of the use of a raw material which is difficult to obtain and the drawback of providing very low reaction yields in the cyclization reaction, as the presence in the phenolic raw material of three equivalent hydroxyl groups leads to the formation of intermolecular methylene bridges in the reaction with the methylene chloride reagent. These by-products depress the yields, make it difficult to recover the desired product and must be sent for special combustion for their disposal.

In conclusion, none of the known processes for the production of 5-hydroxy-MDB has characteristics which make it interesting from an industrial point of view. These processes are, in fact, jeopardized by the use of costly raw materials, when they are not easily available, and also by the use of reagents which are difficult to handle or dangerous and in any case characterized by a heavy environmental impact.

The Applicant has now found that it is possible to overcome all the drawbacks of the processes of the known art for the oxidation of aromatic compounds containing a heterocyclic system, such as benzodioxoles, by means of a direct hydroxylation process of the substrate in the presence of a heterogeneous zeolitic catalyst and hydrogen peroxide.

An object of the present invention therefore relates to a process for the hydroxylation of aromatic compounds containing a heterocyclic system having general formula (I) : 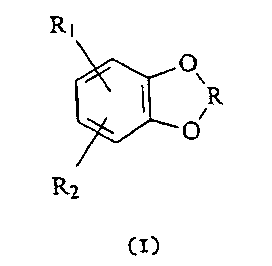 wherein R represents a C₁-C₄ (iso)alkylene radical, whereas R₁ and R₂, the same or different, represent a hydrogen atom or a CH₃ radical or a halogen, or a C₁-C₂ alkoxyl, which comprises directly hydroxylating said compounds having general formula (I) with H₂O₂ in the presence of a zeolitic catalyst having general formula (II):

xTiO₂ · (1-x)SiO₂ (II)

wherein x is a number ranging from 0.0001 to 0.04, preferably from 0.01 to 0.025.

The zeolitic catalyst is known in literature as titanium-silicalite TS-1 and, more specifically, its preparation is described in U.S. patent 4,410,501 and in European patent 200,260. In the present invention the titanium-silicalite catalyst is used with a particle size ranging from 1 to 1000 µm, preferably from 5 to 100 µm or in the form of pellets.

According to the present invention, a substrate which is particularly suitable for being directly hydroxylated is that in which R is a methylene radical, whereas R₁ and R₂ are two hydrogen atoms. This product is 1,3-benzodioxole (MDB) and can be oxidized in position 5 to form 5-hydroxy-MDB.

The hydroxylation reaction can be effected in the presence of one or more solvents or directly in mass, by feeding H₂O₂, optionally diluted with H₂O, to a suspension of titanium-silicalite in the substrate, for example MDB, used in a large excess.

Solvents which can be used are:
- products belonging to the group of aliphatic alcohols, in particular C₁-C₁₀ linear, branched or cyclic alcohols;
- linear, branched or cyclic aliphatic ketones, with a number of carbon atoms ranging from 3 to 12;
- linear, branched or cyclic saturated aliphatic hydrocarbons with a number of carbon atoms ranging from 5 to 12;
- esters selected from dialkyl carbonates wherein the alkyl group contains from 1 to 4 carbon atoms, and esters of carboxylic acid having the formula CH₃-COO-R' wherein R' represents a C₁-C₄ radical;
- linear, branched or cyclic aliphatic ethers, with a number of carbon atoms ranging from 3 to 12;
- aliphatic nitriles having the formula R"-CN, wherein R" represents a C₁-C₄ alkyl radical.

The heterogeneous titanium-silicalite catalyst, known in literature also as TS-1, can be used in batch reactions, in concentrations, with respect to the substrate, varying from 1 to 50% by weight, preferably from 5 to 30%. In this case, the hydrogen peroxide is fed onto a suspension of the TS-1 catalyst in the substrate or onto a suspension consisting of the TS-1 catalyst and a mixture of the substrate with a solvent possibly containing water.

Alternatively, the reaction can also be conveniently carried out in continuous by feeding hydrogen peroxide and the substrate on a layer of TS-1 catalyst or, preferably, by passing the reagents through a fixed bed of TS-1 catalyst in the form of pellets.

The hydrogen peroxide (H₂O₂) reagent can be used with the concentrations of commercial products, for example with concentrations in hydrogen peroxide ranging from 10 to 60% by weight, but can be further diluted to concentrations lower than 10%. In order to obtain high reaction yields, the molar ratio H₂O₂/substrate can vary from 0.01 to 0.5, preferably from 0.1 to 0.3. The reaction can be carried out at a temperature ranging from 10 to 100°C, preferably from 40 to 80°C.

The process, object of the present invention, allows conversion selectivities of the substrate to hydroxylated product to be obtained, in particular of the MDB substrate to 5-hydroxy-MDB, of up to 80% and with conversions up to 30%. The excess substrate which has not reacted and the TS-1 catalyst can be separated from the reaction mixture and recycled for the subsequent syntheses.

Some illustrative but non-limiting examples are provided for a better understanding of the present invention and for its embodiment.

### EXAMPLE 1

30 g of TS-1 catalyst, 150 g of methylisobutylketone, 150 g of acetone and 150 g (1.23 moles) of MDB, are charged into a reactor equipped with a stirrer, thermometer, condenser and heating/cooling jacket. The resulting suspension is heated to 60°C, maintaining the mass under stirring. 84 g of H₂O₂ at 10% (0.25 moles) are then fed in about 4 hours, the temperature of the suspension being maintained at 60°C. The suspension is left under stirring at 60°C for an hour and the organic phase is subsequently analyzed after separation from the TS-1 catalyst.

The following results are obtained from GLC analysis of the organic phase: MDB conversion = 15%; selectivity to 5-hydroxy-MDB = 62%.

### EXAMPLE 2

The same procedure is used as in Example 1 except that only 300 g of acetone are charged instead of the acetone/methylisobutylketone mixture.

The following results are obtained from GLC analysis of the organic phase: MDB conversion = 12%; selectivity to 5-hydroxy-MDB = 58%.

### EXAMPLE 3

The same procedure is used as in Example 1, except that 70 g of H₂O are charged instead of the mixture of solvents and 28 g of H₂O₂ at 30% (0.25 moles), are dosed.

The following results are obtained from GLC analysis of the organic phase: MDB conversion = 10%; selectivity to 5-hydroxy-MDB = 25%.

### EXAMPLE 4

The same procedure is used as in Example 1, except that 33 g of n-hexane are charged instead of acetone.

The following results are obtained from GLC analysis of the organic phase: MDB conversion = 10%; selectivity to 5-hydroxy-MDB = 70%.

### EXAMPLE 5

The same procedure is used as in Example 1, except that 150 g of dimethyl carbonate are charged instead of the acetone/methylisobutylketone mixture.

The following results are obtained from GLC analysis of the organic phase: MDB conversion = 13%; selectivity to 5-hydroxy-MDB = 52%.

### EXAMPLE 6

The same procedure is used as in Example 1, except that 150 g of acetonitrile are charged instead of the acetone/methylisobutylketone mixture.

The following results are obtained from GLC analysis of the organic phase: MDB conversion = 10%; selectivity to 5-hydroxy-MDB = 45%.

### EXAMPLE 7

The same procedure is used as in Example 1, except that 300 g of terbutanol are charged instead of the acetone/methylisobutylketone mixture.

The following results are obtained from GLC analysis of the organic phase: MDB conversion = 15%; selectivity to 5-hydroxy-MDB = 48%.

## Claims

1. A process for the hydroxylation of aromatic compounds containing a heterocyclic system having general formula (I) : 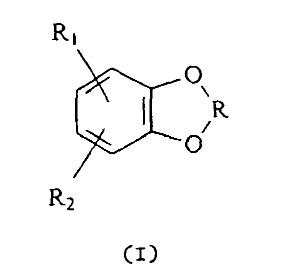 wherein R represents a C₁-C₄ (iso)alkylene radical, whereas R₁ and R₂, the same or different, represent a hydrogen atom or a CH₃ radical, or a C₁-C₂ alkoxyl, which comprises directly hydroxylating said compounds having general formula (I) with H₂O₂ in the presence of a zeolitic catalyst having general formula (II):
xTiO₂ · (1-x)SiO₂ (II)
wherein x is a number ranging from 0.0001 to 0.04, preferably from 0.01 to 0.025.

2. The process according to claim 1, wherein the zeolitic catalyst is used with a particle size ranging from 1 to 1000 µm, preferably from 5 to 100 µm, or in the form of pellets.

3. The process according to claim 1 or 2, wherein in the product having general formula (I), R is a methylene radical whereas R₁ and R₂ are two hydrogen atoms.

4. The process according to any of the previous claims,
wherein the hydroxylation reaction is carried out in the presence of one or more solvents or directly in mass by feeding hydrogen peroxide, optionally diluted with H₂O, to a suspension of catalyst in the substrate.

5. The process according to claim 4, wherein the solvent is selected from:
- aliphatic alcohols, in particular C₁-C₁₀ linear, branched or cyclic alcohols;
- linear, branched or cyclic aliphatic ketones, with a number of carbon atoms ranging from 3 to 12;
- linear, branched or cyclic saturated aliphatic hydrocarbons with a number of carbon atoms ranging from 5 to 12;
- esters selected from dialkyl carbonates wherein the alkyl group contains from 1 to 4 carbon atoms, and esters of carboxylic acid having the formula CH₃-COO-R'
wherein R' represents a C₁-C₄ radical;
- linear, branched or cyclic aliphatic ethers, with a number of carbon atoms ranging from 3 to 12;
- aliphatic nitriles having the formula R"-CN, wherein R" represents a C₁-C₄ alkyl radical.

6. The process according to any of the previous claims, wherein the catalyst is used in batch reactions, in concentrations, with respect to the substrate, ranging from 1 to 50% by weight.

7. The process according to any of the claims from 1 to 5, wherein the reaction is carried out in continuous, feeding hydrogen peroxide and the substrate on a layer of catalyst or by passing the reagents through a fixed bed of catalyst in the form of pellets.

8. The process according to any of the previous claims, wherein the H₂O₂ reagent is used in an aqueous solution with concentrations ranging from 1 to 60% by weight.

9. The process according to any of the previous claims, wherein the molar ratio H₂O₂/substrate varies from 0.01 to 0.5, preferably from 0.1 to 0.3.

10. The process according to any of the previous claims, wherein the oxidation reaction is carried out at a temperature ranging from 10 to 100°C, preferably from 40 to 80°C.

## Revendications

1. Procédé d'hydroxylation de composés aromatiques contenant un système hétérocyclique de formule générale (I) : 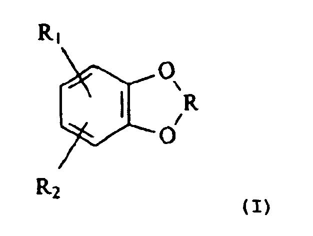 dans laquelle R représente un radical (iso)alkylène en C₁ à C₄, alors que R₁ et R₂, identiques ou différents, représentent un atome d'hydrogène ou un radical CH₃, ou un groupe alcoxyle en C₁ ou C₂, qui comprend l'hydroxylation directe desdits composés de formule générale (I) avec H₂O₂ en présence d'un catalyseur zéolithique de formule générale (II) :
xTiO₂ - (1-x)SiO₂ (II)
dans laquelle x représente un nombre dans la gamme de 0,0001 à 0,04, de préférence de 0,01 à 0,025.

2. Procédé selon la revendication 1, dans lequel le catalyseur zéolithique est utilisé avec une taille de particule dans la gamme de 1 à 1 000 µm, de préférence de 5 à 100 µm, ou sous forme de granulés.

3. Procédé selon la revendication 1 ou 2, dans lequel, dans le produit de formule générale (I), R représente un radical méthylène alors que R₁ et R₂ représentent deux atomes d'hydrogène.

4. Procédé selon l'une quelconque des revendications précédentes, dans lequel la réaction d'hydroxylation est réalisée en présence d'un ou plusieurs solvants ou directement dans la masse en introduisant du peroxyde d'hydrogène, éventuellement dilué avec H₂O, dans une suspension de catalyseur dans le substrat.

5. Procédé selon la revendication 4, dans lequel le solvant est choisi parmi :
- les alcools aliphatiques, en particulier les alcools cycliques, ramifiés ou linéaires en C₁ à C₁₀ ;
- les cétones aliphatiques cycliques, ramifiées ou linéaires comportant un nombre d'atomes de carbone allant de 3 à 12 ;
- les hydrocarbures aliphatiques saturés cycliques, ramifiés ou linéaires comportant un nombre d'atomes de carbone allant de 5 à 12 ;
- les esters choisis parmi les carbonates de dialkyle, dans lesquels le groupe alkyle contient de 1 à 4 atomes de carbone, et les esters d'acide carboxylique de formule CH₃-COO-R', dans laquelle R' représente un radical en C₁ à C₄ ;
- les éthers aliphatiques cycliques, ramifiés ou linéaires comportant un nombre d'atomes de carbone allant de 3 à 12 ;
- les nitriles aliphatiques de formule R" -CN, dans laquelle R" représente un radical en C₁ à C₄.

6. Procédé selon l'une quelconque des revendications précédentes, dans lequel le catalyseur est utilisé dans des réactions discontinues, à des concentrations, par rapport au substrat, dans la gamme de 1% à 50% en poids.

7. Procédé selon l'une quelconque des revendications 1 à 5, dans lequel la réaction est réalisée en continu, en introduisant le peroxyde d'hydrogène et le substrat sur une couche de catalyseur ou en faisant passer les réactifs à travers un lit fixe de catalyseur sous forme de granulés.

8. Procédé selon l'une quelconque des revendications précédentes, dans lequel le réactif H₂O₂ est utilisé en solution aqueuse à des concentrations dans la gamme de 1% à 60% en poids.

9. Procédé selon l'une quelconque des revendications précédentes, dans lequel le rapport molaire H₂O₂/substrat varie de 0,01 à 0,5, de préférence de 0,1 à 0,3.

10. Procédé selon l'une quelconque des revendications précédentes, dans lequel la réaction d'oxydation est réalisée à une température dans la gamme de 10°C à 100°C, de préférence de 40°C à 80°C.

## Patentansprüche

1. Verfahren zur Hydroxylierung aromatischer Verbindungen, die ein heterocyclisches System enthalten und der allgemeinen Formel (I) entsprechen: 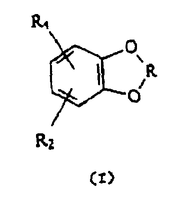 wobei R ein C₁-C₄ (Iso)alkylenradikal darstellt, während R₁ und R₂, die gleich oder verschieden sein können, ein Wasserstoffatom oder ein CH₃₋Radikal oder ein C₁-C₂-Alkoxyl darstellen,
umfassend eine direkte Hydroxylierung der Verbindungen mit der allgemeinen Formel (I) mit H₂O₂ in Gegenwart eines zeolithischen Katalysators mit der allgemeinen Formel (II):
x-TIO₂ • (1-x)SiO₂ (II)
wobei x eine Zahl im Bereich von 0,0001 bis 0,04 ist, vorzugsweise von 0,01 bis 0,025.

2. Verfahren nach Anspruch 1, wobei der zeolichische Katalysator mit einer Partikelgröße im Bereich von 1 bis 1000 µm, vorzugsweise von 5 bis 100 µm, oder in Form von Pellets eingesetzt wird.

3. Verfahren nach Anspruch 1 oder 2, wobei in dem Produkt mit der allgemeinen Formel (I) R ein Methylenradikal ist, während R₁ und R₂ zwei Wasserstoffatome sind.

4. Verfahren nach einem der vorstehenden Ansprüche, wobei die Hydroxylierungsreaktion in Gegenwart eines oder mehrerer Lösungsmittel oder direkt in der Masse durch Zuführen von Wasserstoffperoxid, optional mit H₂O verdünnt, zu einer Suspension des Katalysators in dem Substrat durchgeführt wird.

5. Verfahren nach Anspruch 4, wobei das Lösungsmittel ausgewählt ist aus:
- aliphatischen Alkoholen, insbesondere linearen, verzweigten oder cydischen C₁-C₁₀-Alkoholen;
- linearen, verzweigten oder cyclischen aliphatischen Ketonen mit einer Anzahl von Kohlenstoffatomen im Bereich von 3 bis 12;
- linearen, verzweigten oder cyclischen gesättigten aliphatischen Kohlenwasserstoffen mit einer Anzahl von Kohlenstoffatomen im Bereich von 5 bis 12;
- Estern, die ausgewählt sind aus Dialkylcarbonaten, wobei die Alkylgruppe 1 bis 4 Kohlenstoffatome enthält, und Estern von Carbonsäuren mit der Formel CH₃-COO-R', wobei R' ein C₁-C₄-Radikal darstellt;
- linearen, verzweigten oder cyclischen aliphatischen Ethern mit einer Anzahl von Kohlenstoffatomen im Bereich von 3 bis 12;
- aliphatischen Nitrilen mit der Formel R"-CN, wobei R" ein C₁-C₄-Alkylradikal darstellt.

6. Verfahren nach einem der vorstehenden Ansprüche, wobei der Katalysator bei Chargenreaktionen in Konzentrationen im Bereich von 1 bis 50 Gew.-%, bezogen auf das Substrat, eingesetzt wird.

7. Verfahren nach einem der Ansprüche 1 bis 5, wobei die Reaktion kontinuierlich durchgeführt wird, indem Wasserstof peroxid und das Substrat auf einer Lage des Katalysators geführt werden oder mittels Durchleiten der Reagenzien durch ein Festbett des Katalysators in Form von Pellets.

8. Verfahren nach einem der vorstehenden Ansprüche, wobei das H₂O₂-Reagenz in einer wässrigen Lösung mit Konzentrationen im Bereich von 1 bis 60 Gew.-% eingesetzt wird.

9. Verfahren nach einem der vorstehenden Ansprüche, wobei das Molverhältnis H₂O₂/Substrat 0,01 bis 0,5 beträgt, vorzugsweise 0,1 bis 0,3.

10. Verfahren nach einem der vorstehenden Ansprüche, wobei die Oxidationsreaktion bei Temperaturen im Bereich von 10 bis 100 °C, vorzugsweise von 40 bis 80°C, durchgeführt wird.
